(19) Europäisches Patentamt
European Patent Office
Office européen des brevets

(11) Numéro de publication: **0 128 064**
**B1**

# (12) FASCICULE DE BREVET EUROPÉEN

(45) Date de publication du fascicule du brevet: **02.08.89**

(51) Int. Cl.⁴: **C 07 C 31/02**, C 07 C 29/04

(21) Numéro de dépôt: **84400908.4**

(22) Date de dépôt: **03.05.84**

(54) **Hydratation des oléfines.**

(30) Priorité: **06.05.83 FR 8307649**

(43) Date de publication de la demande:
**12.12.84 Bulletin 84/50**

(45) Mention de la délivrance du brevet:
**02.08.89 Bulletin 89/31**

(84) Etats contractants désignés:
**BE DE FR GB IT NL**

(56) Documents cité:
**EP-A-0 045 618**
**US-A-4 131 750**

**Le dossier contient des informations techniques présentées postérieurement au dépôt de la demande et ne figurant pas dans le présent fascicule.**

(73) Titulaire: **ELF FRANCE Société Anonyme dite:, 137, Rue de l'Université, F-75340 Paris Cedex 07 (FR)**

(72) Inventeur: **Gueguen, Claude, Le Cayet, F-38780 Septème Pont- l'Evêque (FR)**
Inventeur: **Figueras, François, 82 Rue de l'Aiguelongue, F-34100 Montpellier (FR)**
Inventeur: **Fajula, François, 6 Rue du Jeu de Mail, F-34160 Theyran (FR)**

(74) Mandataire: **Boillot, Marc, SOCIETE NATIONALE ELF AQUITAINE Division Propriété Industrielle Tour Elf, F-92078 Paris la Défense Cédex 45 (FR)**

**EP 0 128 064 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne un procédé de préparation d'alcools aliphatiques par hydrafation d'oléfines. Les alcools obtenus sont utiles comme solvants, comme bases pour lubrifiants, ainsi que comme adjuvants des essences.

L'obtention des alcools par hydratation des oléfines en milieu acide est bien connue. Ainsi l'alcool isopropylique est obtenu par hydratation du propylène en milieu acide sulfurique. Cependant, l'utilisation de l'acide sulfurique entraîne des problèmes de corrosion et la reconcentration de l'acide implique une grande consommation d'énergie.

Une amélioration de ce procédé consiste dans l'utilisation de catalyseurs acides solides comme par exemple l'acide phosphorique déposé sur silice ou argile. Ce type de catalyseur est décrit dans le brevet américain 4 299 730. La température très élevée nécessaire pour ces réactions exerce un effet défavorable sur le rendement et la sélectivité, ce qui pour l'instant empèche le développement de ces techniques.

L'utilisation de catalyseurs de type zéolithe a également été envisagé. Selon le brevet français 2 281 344 on utilise un zéolithe Y contenant des cations de chrome et de terres rares. Ces cations assurent une certaine acidité au catalyseur.

D'autres procédés utilisent les zéolithes sous forme protonée. Ainsi le brevet américain 4 214 107 préconise l'utilisation d'une zéolithe synthétique, dénommée ZSM-5 sous forme protonée.

Le brevet japonais 7 245 323 décrit l'utilisation de la mordénite et de la faujasite également sous leur forme protonée.

Cependant, ces zéolithes ne sont pas des catalyseurs très actifs et ne permettent pas d'atteindre des conversions importantes. Les oléfines peu réactives, comme les n-butènes ne réagissent pas en présence de ces zéolithes.

On a trouvé maintenant un procédé de préparation d'alcools aliphatiques par hydratation des oléfines correspondantes par traitement à une température de 100 à 400°C une pression de 20 à 100 bars, une vitesse spatiale liquide de la charge (VVH) de 0,2 à 5 $m^3/m^3/h$, le rapport molaire eau/oléfine étant compris entre 1 et 8 en présence d'un système catalytique à base d'un silicoaluminate cristallin zéolithique du type de l'offretite et en l'absence d'acide fort. L'offretite utilisée peut être soit l'offretite naturelle soit une offretite synthétique qui possède une structure cristalline identique à l'offretite naturelle et un rapport molaire silice/alumine compris entre 2 et 50.

L'offretite naturelle est bien décrite par J.M. BENNETT et J.A. GARD (Nature 214 1005 (1967). Elle ne comporte pas de défauts cristallins et se distingue ainsi très nettement de l'érionite et de la zéolithe T.

La structure de l'offretite se définit comme un ensemble de canaux cylindriques, rectilignes et parallèles présentant une ouverture de diamètre 0,63 nm environs. Ces canaux sont limités par des empilements de prismes hexagonaux et de cages de petites dimensions.

Les empilements sont eux-mêmes reliés par des cages présentant des ouvertures à huit cotés accessibles à des molécules de diamètre critique 0,5 nm.

L'offretite appartient au groupe 2 de la classification de MEIER (W.M. MEIER Molecular Sieves, Society of Chemical Industry, London 1968) universellement admise et le spectre de diffraction des rayons X possède les pics significatifs donnés dans le tableau 1 suivant:

**Tableau I**

| khl | d(A) | $I_r$ | hkl | d(A) | $I_r$ | hkl | (A) | $I_r$ |
|---|---|---|---|---|---|---|---|---|
| 100 | 11.50 | 100 | 310 | 3.190 | 17 | 311 | 2.126 | 4 |
| 110 | 6.64 | 20 | 311 | 2.942 | 3 | 303 | 2.110 | 2 |
| 200 | 5,76 | 35 | 400 | 2.880 | 64 | 421 | 2.091 | 2 |
| 201 | 4.581 | 4 | 212 | 2.858 | 15 | 510 | 2.068 | 2 |
| 210 | 4.352 | 59 | 401 | 2.693 | 3 | 511 | 1.995 | 2 |
| 300 | 3.837 | 43 | 320 | 2.642 | 4 | 502 | 1.967 | 2 |
| 211 | 3.774 | 11 | 410 | 2.510 | 20 | 430 | 1.893 | 1 |
| 102 | 3.600 | 3 | 500 | 2.300 | 5 | 520 | 1.844 | 3 |
| 220 | 3.322 | 22 | 420 | 2.177 | 2 | | | |

$I_R$ (intensité relative): $I/I_O \times 100$

Cette zéolithe développe une acidité plus forte que celle des zéolithes Y. Grace à cette acidité et l'accessibilité de ses canaux elle est un catalyseur très efficace.

La maille élémentaire de l'offretite naturelle est représentée par la formule chimique typique:

$$(M_1M_2...)_2\ Al_4\ Si_{14}\ O_{36}\ 14H_2O.$$

La synthèse de la zéolithe permet de modifier les teneurs en silice et alumine.

La synthèse des zéolites consiste à faire cristalliser entre 0 et 300°C des solutions alcalines sursaturées de gels d'alumine et de silice fraichement précipités. Selon le brevet américain 3 947 482 de Grace, le réseau

cristallin est formé autour d'un agent de nucléation organique, en général un ammonium quaternaire.

Une synthèse sans agent de nucléation est décrite par le brevet américain 4 093 699 de Zeochem. Corporation. Dans ce procédé les rapports molaires des composants ainsi que les paramètres opératoires sont bien définis et doivent être scrupuleusement respectés.

Les offretites synthétiques selon l'invention peuvent être définies par la formule:

$$(M_1M_2...)_2\,(Al_2O_3),\,(SiO_2)_y$$

ou y compris entre 2 et 50 et de préférence entre 5 et 15 $M_1$ et $M_2$ sont choisis dans le groupe des cations I et II de la classification périodique.

La cations originaux de la zéolithe peuvent être échangés à 70 % avec une solution d'acétate d'ammonium selon les techniques bien connues. Le catalyseur obtenu est calciné sous air à 500° C pendant 5 heures ou plus. On peut également remplacer les cations originaux par le proton, par des ions métalliques, comme le potassium et le chrome ou encore par les mélanges de cations. Les méthodes d'échange couramment appliquées sont celles décrites dans l'ouvrage "Zéolithe Chemistry and Catalysis" de J.A. Rabu, A.C.S. Monograph 111 (Washington, 1977).

Il peut être intéressant d'incorporer l'offretite à une matière active ou inerte, comme par exemple les argiles, de l'alumine ou encore des compositions binaires silice-alumine, silice-magnésie.

Parmi les oléfines conviennent tout particulièrement les oléfines à bas poids moléculaire ayant jusqu'à 5 atomes de carbone, comme l'éthylène, le propylène, le n-butène-1, le cis et trans n-butène-2, l'isobutène le butadiène et les pentènes. Il est également possible d'utiliser des coupes renfermant un mélange de ces oléfines, comme par exemple la coupe $C_4$, mélange de butane, des butènes et de butadiène, ou la coupe $C_5$, mélange de méthylbutanes et de n-pentènes.

Les oléfines à chaîne droite comme les n-pentènes, n-butènes et le propylène sont normalement peu réactifs et leur hydratation est très difficile. Les catalyseurs très actifs selon l'invention permettent l'hydratation des n-pentènes et n-butènes seuls ou en mélange avec les isopentènes, l'isobutène et le propylène.

La charge hydrocarbonée est mélangée avant d'entrer dans le réacteur avec la vapeur d'eau. Le rapport molaire eau sur oléfine varie entre 1 à 8.

La température de la réaction se situe entre 100 et 400° C. La température choisie dépend de la réactivité de l'oléfine. L'étude de l'équilibre thermodynamique montre que la formation de l'alcool est privilégiée à basse température. On a donc intérêt de choisir la température la plus basse compatible avec un niveau de conversion suffisante.

La pression peut varier entre 20 et 100 bars de pression totale. La vitesse spatiale liquide de la charge ou VVH varie généralement entre 0,2 à 5 $m^3/m^3/h$. Le procédé est illustré par les exemples ci-après donnés à titre non limitatif.

**Exemples**

Le tableau 1 résume les caractéristiques des catalyseurs utilisés dans les exemples.

Les échanges de cations sont faits sur 1 gramme de zéolithe dans 500 $cm^3$ d'eau. On ajoute une solution aqueuse contenant une concentration calculée de sel métallique. L'échange a lieu sous agitation à la température ambiante. Ensuite le catalyseur est filtré, lavé à l'eau distillée, séché à 100° C pendant 12 heures, broyé et activé. Pour l'activation le catalyseur est déposé en lit mince et calciné sous flux d'air sec.

**Exemple 1**

Le tableau 2, résume les résultats de l'hydratation de l'isobutène en terbutanol en présence de différentes offretites. Les catalyseurs 2 et 3 ont une formule identique, mais font partie de deux lots de fabrication différents. Les résultats prouvent la bonne reproductibilité du procédé.

**Exemple 2**

Le tableau 3 résume les résultats de l'hydratation d'un mélange de n-butènes et d'isobutène. Les n-butènes sont principalement du 2-butène cis et trans.

**Exemple 3**

Nous avons résumé dans le tableau 4 l'influence des paramètres expérimentaux sur l'hydratation des n-butènes. La conversion des n-butènes nécessite une température et une pression plus élevée que l'isobutène.

**Exemples 4 et 5**

Les tableaux 5 et 6 résument les résultats de l'hydratation des n-butènes des n-pentènes et les isopentènes.

**Tableau 1:** Catalyseurs

| N° | Nom | Si/Al | Taux d'échange des cations initiaux (%) |
|---|---|---|---|
| 1 | offretite KH | 3,7 | 66 |
| 2 | offretite KH Cr | 3,7 | 70 |
| 3 | offretite KG Cr | 3,7 | 70 |
| 4 | offretite KH Ce | 3,7 | 70 |
| 5 | offretite K Cr | 3,7 | 12 |
| 6 | offretite KH | 8,5 | 70 |
| 7 | offretite KH Cr | 3 | 80 |
| 8 | offretite KH | 3 | 70 |
| 9 | offretite KH | 3,9 | 70 |
| 10 | offretite KH | 3 | 70 |
| 11 | offretite KH | 3 | 66 |
| 12 | idem à 11 mais désaluminés | 3,8 | 83 |
| 13 | idem à 11 mais désaluminés et calciné | 4,96 | 86 |

K = potassium
Cr = chrome
Ce = cerium

**Tableau 2:** Réactions de l'isobutène à 200° C

| N° | Catalyseur Nom | Température °C | $H_2O$/isobutène mole/mole | Pression bar | Conversion en terbutanol % molaire | Rendement en terbutanol $10^{-3}$ moles $g^{-1}h^{-1}$ |
|---|---|---|---|---|---|---|
| 2 | HK Cr offretite | 200 | 5 | 41 | 9,05 | 19,4 |
| 3 | HK Cr offretite | 200 | 5 | 41 | 9 | 19,2 |
| 5 | K Cr offretite | 200 | 4,4 | 45 | 4,7 | 10 |
| 7 | KH Cr offretite | 200 | 5 | 45 | 9 | 19,6 |

**Tableau 3:** Réactions du melange m-butènes-isobutène à 200° C sur offretite

| N° | Catalyseur Nom | Température C° | Pression bar | $H_2O$/oléfine mole/mole | n-butène/ isobutène mole/mole | conversion % molaire isobutène | conversion % molaire n-butène | Rendement en alcool correspondant $10^{-3}$ moles $g^{-1}h^{-1}$ isobutène | Rendement en alcool correspondant $10^{-3}$ moles $g^{-1}h^{-1}$ n-butène |
|---|---|---|---|---|---|---|---|---|---|
| 2 | HKCr offretite | 200 | 45 | 4,4 | 3 | 6,7 | 0,2 | 3,6 | 0,4 |

**Tableau 4:** Influence des paramètres experimentaux dans l'hydratation des n-butènes

| N° | Catalyseur nom | Température °C | $H_2O$/butène mole/mole | Pression bar | Conversion % molaire | Rendement $10^{-3}$ moles $g^{-1}h^{-1}$ |
|---|---|---|---|---|---|---|
| 1 | KH offretite | 200 | 44 | 45 | traces | |

| | | | | | | |
|---|---|---|---|---|---|---|
| 1 | KH offretite | 200 | 6 | 46 | traces | |
| 1 | KH offretite | 250 | 4,4 | 65 | 0,9 | 1,9 |

**Tableau 5:** Hydratation des n-butènes sur offretite KH - Influence de la structure de l'offretite

| n° | Catalyseur nom | Température °C | $H_2O$/n-butène mole/mole | Pression bar | Rendement exprimé en % de l'équilibre thermodynamique | Rendement en butanol $10^{-3}$ moles $g^{-1}h^{-1}$ |
|---|---|---|---|---|---|---|
| 1 | offretite KF | 250 | 4,5 | 65 | 28 | 2,63 |
| 8 | offretite KH | 250 | 4,5 | 65 | 20 | 1,88 |
| 9 | offretite KH | 250 | 4,5 | 65 | 33 | 3,1 |
| 10 | offretite KH | 250 | 4,5 | 65 | 16 | 1,5 |
| 11 | offretite KH | 250 | 4,5 | 65 | 19 | 1,85 |
| 12 | offretite desaluminé | 250 | 4,5 | 65 | 40 | 3,7 |
| 13 | offretite désaluminé puis calciné | 250 | 4,5 | 65 | 64 | 6,02 |

**Tableau 6:** Influence des paramètres expérimentaux dans l'hydratation des n-pentènes et isopentènes

| N° | Catalyseur Nom | Oléfine | Temp. °C | $H_2O$/pentène mole/mole | Pression bar | Conversion % molaire | Rendement exprimé en % de l'équilibre thermodynamique | Rendement en en alcool $10^{-3}$ moles $g^{-1}h^{-1}$ |
|---|---|---|---|---|---|---|---|---|
| 8 | offretite KH | methyl 2-pentène-2 | 200 | 4,5 | 35 | 4,46 | 80 | 8,42[a] |
| 8 | offretite KH | methyl 2-pentène-2 | 250 | 4,5 | 67,5 | 4,42 | 85 | 8,36[a] |
| 8 | offretite KH | pentène-2 cis et trans | 250 | 4,5 | 65 | traces | - | - |
| 8 | offretite KH | pentène-2 cis et trans | 300 | 4,5 | 115 | 3,8 | 90 | 7,04[b] |

a = methyl-2-pentanol-2
b = pentanol-2 et pentanol-3

## Revendications

1. Procédé de préparation d'alcools aliphatiques par hydratation des oléfines correspondantes en présence d'un système catalytique à base d'un silicoaluminate cristallin zéolithique du type offretite, caractérisé en ce qu'on opère en l'absence d'acide fort, à une température de 100 à 400°C et sous une pression de 20 à 100 bars avec une vitesse spatiale liquide de la charge (VVH) de 0,2 à 5 $m^3/m^3/h$, le rapport molaire eau/oléfine étant compris entre 1 et 8.

2. Procédé selon la revendication 1, caractérisé en ce que la zéolithe utilisée est de l'offretite naturelle.

3. Procédé selon la revendication 1 caractérisé en ce que la zéolithe utilisée est une offretite synthétique qui possède une structure cristalline identique à celle de l'offretite naturelle et répond à la formule chimique $(M_1M_2...)_2\,(Al_2O_3)\,(SiO_2)_y$, où y est compris entre 2 et 50, $M_1$ et $M_2$ sont choisis dans le groupe des cations I et II de la classification périodique.

4. Procédé selon la revendication 1 caractérisé en ce qu'on échange au moins partiellement les cations originaux de l'offretite avec des ions métalliques, des ions ammoniums, des protons, pris séparément ou en mélange.

5. Procédé selon les revendications 1 à 4 caractérisé en ce que les ions sodium et potassium de l'offretite sont échangés à 70 % avec une solution d'acétate d'ammonium et que le catalyseur ainsi obtenu est traité 5 heures ou plus à 500°C en présence d'air avant d'être utilisé dans la réaction.

6. Procédé selon les revendications 1 à 4 caractérisé en ce que les ions potassium et sodium de l'offretite sont échangés avec un sel de chrome.

7. Procédé selon les revendications 1 et 3 caractérisé en ce que le rapport molaire silice/alumine est compris entre 5 et 15.

8. Procédé selon les révendications 1 à 7 caractérisé en ce qu'il est appliqué à des oléfines ayant jusqu'à cinq atomes de carbone.

9. Procédé selon les revendications 1 à 8 caractérisé en ce qu'il est appliqué à des coupes pétrolières composés d'hydrocarbure saturés et insaturés à quatre atomes de carbone.

10. Procédé selon la revendication 9 caractérisé en ce qu'il est appliqué aux n-butènes.

11. Procédé selon les revendications 1 à 8 caractérisé en ce qu'il est appliqué à des coupes pétrolières composés d'hydrocarbures saturés et insaturés à cinq atomes de carbone.

12. Procédé selon la revendication 11 caractérisé en ce qu'il est appliqué aux n-pentènes.

**Patentansprüche**

1. Verfahren zur Herstellung von aliphatischen Alkoholen durch Hydratisierung der entsprechenden Olefine in Gegenwart eines katalytischen Systems auf der Basis eines kristallinen zeolithischen Aluminiumsilikats vom Typ Offretit, dadurch gekennzeichnet, daß man in Abwesenheit einer starken Säure, bei einer Temperatur von 100 bis 400°C und unter einem Druck von 20 bis 100 bar mit einer auf Flüssigkeit bezogenen Raumgeschwindigkeit der Charge (VVH) von 0,2 bis 5 $m^3/m^3/h$, arbeitet, wobei das Molverhältnis Wasser/Olefin zwischen 1 und 8 beträgt.

2. Verfahren nach Anspruch 1, dadurch gekennzeichnet, daß der verwendete Zeolith natürlicher Offretit ist.

3. Verfahren nach Anspruch 1, dadurch gekennzeichnet, daß der verwendete Zeolith ein synthetischer Offretit ist, die gleiche kristalline Struktur hat, wie natürlicher Offretit, und der chemischen Formel $(M_1M_2...)_2$ $(Al_2O3)$ $(SiO_2)_y$ entspricht, worin y zwischen 2 und 50 bedeutet, und $M_1$ und $M_2$ aus Kationen der Gruppe I und II des Periodensystems ausgewählt sind.

4. Verfahren nach Anspruch 1, dadurch gekennzeichnet, daß man die ursprünglichen Kationen des Offretits wenigstens teilweise mit Metallionen, Ammoniumionen und Protonen einzeln oder gemischt austauscht.

5. Verfahren nach den Ansprüchen 1 bis 4, dadurch gekennzeichnet, daß die Natriumionen und Kaliumionen des Offretits zu 70 % mit einer Ammoniumacetatlösung ausgetauscht werden, und daß der so erhaltene Katalysator 5 Stunden oder länger, bei 500°C in Anwesenheit von Luft behandelt wird, bevor er in der Reaktion verwendet wird.

6. Verfahren nach den Ansprüchen 1 bis 4, dadurch gekennzeichnet, daß die Kaliumionen und Natriumionen des Offretits mit einem Chromsalz ausgetauscht werden.

7. Verfahren nach den Ansprüchen 1 und 3, dadurch gekennzeichnet, daß das Molverhältnis Siliziumoxid /Aluminiumoxid zwischen 5 und 15 beträgt.

8. Verfahren nach den Ansprüchen 1 bis 7, dadurch gekennzeichnet, daß es bei Olefinen mit bis zu 5 Kohlenstoffatomen angewendet wird.

9. Verfahren nach den Ansprüchen 1 bis 8, dadurch gekennzeichnet, daß es bei Erdölfraktionen aus gesättigten und ungesättigten Kohlenwasserstoffen mit 4 Kohlenstoffatomen angewendet wird.

10. Verfahren nach Anspruch 9, dadurch gekennzeichnet, daß es bei n-Butenen angewendet wird.

11. Verfahren nach den Ansprüchen 1 bis 8, dadurch gekennzeichnet, daß es bei Erdölfraktionen aus gesättigten und ungesättigten Kohlenwasserstoffen mit 5 Kohlenstoffatomen angewendet wird.

12. Verfahren nach Anspruch 11, dadurch gekennzeichnet, daß es bei n-Pentenen angewendet wird.

**Claims**

1. Process for the preparation of aliphatic alcohols by the hydration of corresponding olefins in the presence of a catalytic system based on a zeolithic crystalline silicoaluminate of the offretite type, characterised in that the process is carried out in the absence of strong acid, at a temperature from 100°C to 400°C and under a pressure of from 20 to 100 bars at a liquid space velocity of charge (VVH) of from 0.2 to $5m^3/m^3/h$, the water/olefin molar ratio being between 1 and 8.

2. Process according to Claim 1, characterised in that the zeolite used is natural offretite.

3. Process according to Claim 1 characterised in that the zeolite used is a synthetic offretite which has a crystaline structure identical to that of natural offretite and which conforms to the chemical formula $(M_1\ M_2...)_2$ $(Al_2O_3)$ $(SiO_2)_y$, where y is between 2 and 50, $M_1$ and $M_2$ are selected from the group of cations of groups I and II of the periodic table.

4. Process according to Claim 1 characterised in that the original offretite cations are at least partially exchanged with metal ions, ammonium ions, protons, either taken separately or as a mixture.

5. Process according to Claims 1 to 4 characterised in that 70 % of the offretite sodium and potassium ions are exchanged with an ammonium acetate solution and in that the catalyst thus obtained is treated for 5 hours or more at 500°C in the presence of air before being used in the reaction.

6. Process according to Claims 1 to 4 characterised in that the sodium and potassium ions of the offretite are

exchanged with a chromium salt.

7. Process according to Claims 1 to 3 characterised in that the silica/alumina molar ratio is between 5 and 15.

8. Process according to Claims 1 to 7 characterised in that it is applied to olefins having up to 5 carbon atoms.

9. Process according to Claims 1 to 8 characterised in that it is applied to petroleum fractions comprising saturated and unsaturated hydrocarbons having 4 carbon atoms.

10. Process according to Claim 9 characterised in that it is applied to n-butenes.

11. Process according to Claims 1 to 8 characterised in that is applied to petroleum fractions comprising saturated and unsaturated hydrocarbons having 5 carbon atoms.

12. Process according to Claim 11 characterised in that it is applied to n-pentenes.